# EUROPÄISCHE PATENTSCHRIFT

(11) **EP 1 191 012 B1**
(45) Veröffentlichungstag und Bekanntmachung des Hinweises auf die Patenterteilung: **17.01.2007**
(21) Anmeldenummer: 01121625.6
(22) Anmeldetag: 12.09.2001
(51) Int. Cl.: C07C 45/41, C07C 47/55

(54) **Verfahren zur Herstellung von Fluor enthaltenden Benzaldehyden**
Process for the preparation of fluorine containing benzaldehydes
Procédé de préparation d'aldéhydes benzéniques fluorés

(30) Priorität: 25.09.2000 DE 10047293
(43) Veröffentlichungstag der Anmeldung: 27.03.2002
(73) Patentinhaber: Saltigo GmbH, 51369 Leverkusen (DE)
(72) Erfinder: Baumann, Käthe, Dr., 42109 Wuppertal (DE); Marhold, Albrecht, Dr., 51373 Leverkusen (DE)
(74) Vertreter: Wichmann, Birgid

(56) Entgegenhaltungen:
- EP-A- 0 071 065
- BURGER; HORNBAKER: "Some aromatic trifluoromethyl derivaives. The Rosenmund Reduction of trifluoromethylbenzoic acids" J. ORG. CHEM., Bd. 18, 1953, Seiten 192-194, XP001038078
- "Organic Reactions vol. IV, 362-377" 1979 , ROBERT E. KRIEGER PUBLISHING COMPANY , NEW YORK (US) XP002185424 * Seite 366, Zeile 12 - Seite 368, Zeile 18 *

## Beschreibung

Die vorliegende Erfindung betrifft ein Verfahren zur Herstellung von Fluor enthaltenden Benzaldehyden durch Reduktion der entsprechenden aromatischen Säurechloride.

Fluorierte Benzaldehyde sind wichtige Bausteine für Wirkstoffe im Pharmabereich. Sie können auch durch Reduktion zu den entsprechenden Benzylalkoholen umgewandelt werden, die ebenfalls für Wirkstoffe im Pharmabereich eine breite Anwendung finden.

Es sind bereits Verfahren zur Herstellung von Fluor enthaltenden Benzaldehyden bekannt. So wurden fluorierte Benzoylchloride durch eine Rosenmund-Reaktion (siehe Org. React. Vol. 4, 362) in Sulfolan als Lösungsmittel zu den entsprechenden Benzaldehyden reduziert (EP-A 71 065). Es werden auf diese Weise hohe Ausbeuten erhalten, aber durch den Einsatz eines Lösungsmittels besitzt das Verfahren den Nachteil niedriger Raum-Zeit-Ausbeute und höherer Materialkosten. Es wird außerdem, insbesondere im Falle des 3,5-Bis-(trifluormethyl)-benzoylchlorids eine gesteigerte Überhydrierung des Benzoylchlorids zum entsprechenden Benzol beobachtet.

Speziell zur Herstellung von 3,5-Bis-(trifluormethyl)-benzaldehyd gibt es mehrere bekannte Synthesewege die sich alle nicht für eine Herstellung im größeren Maßstab eignen. So wurde das entsprechende Brombenzol mit Butyllithium und N,N-Dimethylformamid umgesetzt (J. Med. Chem. 16, 1399 (1973) und Chem. Ber. 129, 233 (1996)). Dieses Verfahren erfordert wegen der Handhabung selbstentzündlicher lithiumorganischer Verbindungen besonders hohen sicherheitstechnischen Aufwand.

Ähnlich hierzu ist die Grignard-Reaktion des entsprechenden Brombenzols mit Magnesium und Orthoameisensäuretriethylester (Eur. J. Med. Chem. Chim. Ther. 14, 411 (1979)), die einen ähnlich hohen sicherheitstechnischen Aufwand für die Handhabung magnesiumorganischer Verbindungen erfordert.

Der technisch schwer zugängliche 3,5-Bis-(trifluormethyl)-benzylalkohol wurde auch schon mit Pyridiniumdichromat in mäßiger Ausbeute oxidiert (J. Amer. Chem. Soc. 107, 2442 (1985)). Dabei entstehen toxische chromhaltige Abfälle, die in aufwendiger Weise entsorgt werden müssen.

Eine Stephen-Reduktion des entsprechenden Nitrils mit Zinn(II)chlorid/Chlorwasserstoffgas (J. Chem. Soc. Perkin Trans. 2, **1987**, 639) ergibt als Abfallprodukt stöchiometrische Mengen toxischer Zinnsalze.

Eine Reduktion des Benzoylchlorids ist auch mit Tri-tert.-butoxy-lithium-aluminium-hydrid in Diglyme beschrieben (J. Med. Chem. 15, 775 (1972)). Aluminiumhydride greifen nach unserer Erfahrung Trifluormethylgruppen an. Als weiterer Nachteil ist das Anfallen stöchiometrischer Mengen von Aluminiumsalzen zu sehen, die entsorgt werden müssen.

Es wurde nun ein Verfahren zur Herstellung von Fluor enthaltenden Benzaldehyden der Formel 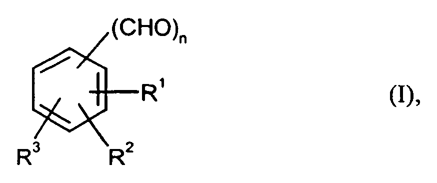 in der
- n: für 1 oder 2 steht und
- R¹, R² und R³: unabhängig voneinander jeweils für Wasserstoff, Fluor, Chlor, Brom, C₁-C₆-Alkyl, C₁-C₆-Fluoralkyl, C₁-C₆-Fluoralkoxy oder C₁-C₆-Fluoralkylthio stehen,
wobei mindestens einer der Reste R¹ bis R³ für Fluor oder einen Fluor enthaltenden Rest steht und nicht mehr als zwei der Reste R¹ bis R³ für Brom, C₁-C₆-Fluoralkoxy und/oder C₁-C₆-Fluoralkylthio stehen,
gefunden, das dadurch gekennzeichnet ist, dass man ein aromatisches Säurechlorid der Formel 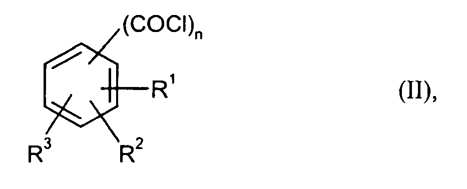 in der die verwendeten Symbole die bei Formel (I) angegebene Bedeutung haben,
mit Wasserstoff in Gegenwart eines geträgerten Palladium-Katalysators und eines Katalysator-Moderators in Abwesenheit eines Lösungsmittels umsetzt.

Soweit die Reste R¹ bis R³ C₁-C₆-Fluoralkyl, C₁-C₆-Fluoralkoxy oder C₁-C₆-Fluoralkylthio bedeuten, kann es sich um monofluorierte, polyfluorierte oder perfluorierte C₁-C₆-Alkyl-, C₁-C₆-Alkoxy- oder C₁-C₆-Fluoralkylthioreste handeln.

Vorzugsweise stehen die Reste R¹ bis R³ unabhängig voneinander für H, F, Cl, Br, CH₃, C₂H₅, CF₃, CF₂CH₃, C₂F₅, OCF₃ oder SCF₃, wobei mindestens einer der Reste R¹ bis R⁴ für F, CF₃, CF₂CH₃, C₂F₅, OCF₃ oder SCF₃ und höchstens einer der Reste R¹ bis R⁴ für Brom, OCF₃ oder SCF₃ steht.

Sofern die Reste R¹ bis R³ von Wasserstoff verschieden sind und nur eine COCl-Gruppe vorhanden ist, stehen sie bevorzugt in 3-, 4- und/oder 5-Stellung zur COCI-Gruppe.

Besonders bevorzugt werden Mono-, Di- und Trifluorbenzoylchloride, Mono- und Bis-trifluormethylbenzoylchloride, Monotrifluormethoxybenzoylchloride und Monochlor- und Monobromtrifluormethylbenzoylchloride in das erfindungsgemäße Verfahren eingesetzt und die entsprechenden Fluor enthaltenden Benzaldehyde hergestellt.

Der Wasserstoff kann beispielsweise mit Drucken im Bereich 0,5 bis 3 bar angewendet werden. Bevorzugt ist seine Anwendung unter atmosphärischem Druck. Das Wasserstoffgas kann z.B. mittels eines Rohres oder einer Fritte in das Reaktionsgemisch eingeleitet werden. Es kann auch auf die Mischung, d.h. in den Raum oberhalb der Mischung, geleitet werden. Bevorzugt ist die Einleitung ins Reaktionsgemisch.

Als Trägermaterial für den geträgerten Palladium-Katalysator kommen z.B. Kohlen, Aluminiumoxide, Silikate, Kieselsäure und Bariumsulfat in Frage. Bevorzugt sind Kohlen und Bariumsulfat. Die geträgerten Palladium-Katalysatoren können z.B. 1 bis 10 Gew.-% Palladium enthalten. Das Gewichtsverhältnis von geträgertem Palladium-Katalysator zum eingesetzten aromatischen Säurechlorid kann beispielsweise bei 1:2 bis 1:1000 liegen. Vorzugsweise liegt es bei 1:5 bis 1:500.

Als Katalysator-Moderatoren kommen beispielsweise organische Schwefelverbindungen in Frage. Bevorzugt sind Thiophenol, Thioanisol, Thioharnstoff, Sulfolan und Chinolin-Schwefel-Komplexe. Besonders bevorzugt sind Chinolin-Schwefel-Komplexe, die beispielsweise wie in Org. Reactions, Vol. 4, 362 beschrieben oder entsprechend dem vorliegenden Beispiel 1 oder analog dazu erhalten werden können.

Das Gewichtsverhältnis von Katalysator-Moderator zu geträgertem Palladium-Katalysator kann beispielsweise 1:1 bis 1:500 betragen. Vorzugsweise liegt es bei 1:10 bis 1:200.

Den Katalysator-Moderator kann man z.B. zusammen mit dem aromatischen Säurechlorid und dem Katalysator vorlegen. Man kann auch zunächst den geträgerten Palladiumkatalysator mit dem Katalysator-Moderator in Kontakt bringen, gegebenenfalls in Gegenwart eines Hilfsstoffs und dann den so mit dem Moderator versehenen Katalysator in das erfindungsgemäße Verfahren einsetzen.

Nach beendeter Umsetzung kann man den Katalysator abtrennen, z.B. durch Filtration, und im nächsten Ansatz wiederverwenden. Man kann diese Wiederverwendung bis zu 5 mal und mehr wiederholen. Wiederverwendeter Katalysator kann i.a. ohne erneuten Zusatz von Katalysator-Moderator eingesetzt werden.

Bei dem Hilfsstoff kann es sich z.B. um kleine Mengen eines aromatischen Kohlenwasserstoffs, eines Halogenkohlenwasserstoffs, eines Halogenaromaten, eines aprotischen Amids, eines gegegebenenfalls cyclischen Ethers oder eines Sulfons handeln. Unter dem Begriff "kleine Mengen" werden z.B. Mengen von bis zu 2,5 ml pro 100 g eingesetztes aromatisches Säurechlorid verstanden. Vorzugsweise liegt diese Menge bei 0,02 bis 0,5 ml pro 100 g eingesetztes aromatisches Säurechlorid.

Der Hilfsstoff kann nicht nur zum besseren in Kontakt bringen von geträgertem Palladium-Katalysator und Katalysator-Moderator dienen, sondern beispielsweise auch zum Anschlämmen eines bereits Katalysator-Moderator enthaltenden geträgerten Palladium-Katalysators vor der Zugabe von aromatischem Säurechlorid.

Die erfindungsgemäße Umsetzung führt man bei Temperaturen durch, bei denen das Edukt flüssig ist, beispielsweise bei Temperaturen im Bereich 20 bis 200°C. Wenn ein Edukt einen Schmelzpunkt von über 20°C aufweist ist der Schmelzpunkt des Edukts die niedrigst mögliche Umsetzungstemperatur. Wenn ein Edukt bei Normaldruck unter 200°C siedet ist gegebenenfalls unter erhöhtem Druck zu arbeiten, so dass das Edukt in flüssigem Zustand bleibt. Es ist auch vorteilhaft, die erfindungsgemäße Umsetzung bei solchen Temperaturen und Drucken durchzuführen, bei denen das jeweilige Produkt flüssig ist. Im allgemeinen kann man bei Temperaturen im Bereich von 70 bis 130°C bei Normaldruck arbeiten. Besonders bevorzugte Umsetzungstemperaturen sind solche im Bereich von 80 bis 120°C.

Die erfindungsgemäße Umsetzung kann man z.B. durchführen, indem man ein aromatisches Säurechlorid, einen geträgerten Palladium-Katalysator, der einen Katalysator-Moderator enthält und gegebenenfalls eine kleine Menge Hilfsstoff vorlegt und unter Zuleitung von Wasserstoff die Reaktionsbedingungen einstellt. Man kann auch ein aromatisches Säurechlorid, einen geträgerten Palladium-Katalysator, einen Katalysator-Moderator und gegebenenfalls eine kleine Menge Hilfsstoff vorlegen und unter Zuleitung von Wasserstoff die Reaktionsbedingungen einstellen.

Die Umsetzung ist beendet, wenn die Reaktionsabgase nicht mehr sauer reagieren. Die Aufarbeitung des gegebenenfalls nach Abkühlung und Druckentspannung vorliegenden Reaktionsgemisches kann auf verschiedene Weise erfolgen, beispielsweise indem man den hergestellten Fluor enthaltenden Benzaldehyd, gegebenenfalls bei vermindertem Druck, direkt aus dem Reaktionsgemisch abdestilliert.

Man kann auch zunächst den Katalysator abtrennen, z.B. durch Filtration, und dann das Produkt durch Destillation, gegebenenfalls unter vermindertem Druck, aus dem Filtrat isolieren. Dabei kann als Vorlauf gegebenenfalls eine kleine Menge überhydriertes Produkt (= Benzolderivat) und/oder eine kleine Menge des gegebenenfalls eingesetzten Hilfsstoffs anfallen.

Man kann häufig auch das nach Abtrennung des Katalysators vorliegende rohe Produkt direkt weiterverwenden, z.B. zur Herstellung fluorierter Benzylakohole durch Reduktion.

Mit dem erfindungsgemäßen Verfahren gelingt es, fluorierte Benzaldehyde der Formel (I) in höheren Raum-Zeit-Ausbeuten, ohne Lösungsmittel und mit weniger Überhydrierung bis zur Benzol-Stufe herzustellen als bisher, wobei keine toxischen Abfälle erhalten werden und kein besonderer sicherheitstechnischer Aufwand nötig ist.

### Beispiele

### Beispiel 1

60 g frisch destilliertes Chinolin und 10 g Schwefel wurden für 5 Stunden auf Rückfluss erhitzt und gerührt. Die erkaltete Mischung wurde mit 700 ml Toluol verdünnt. Es wurde so eine Lösung eines Chinolin-Schwefel-Komplexes erhalten, die pro ml 100 mg des Komplexes enthielt.

2,5 g 5 gew.-%iges Palladium auf Bariumsulfat, 0,25 ml der Komplexlösung und 250 g 3,5-Bis-(trifluormethyl)-benzoylchlorid wurden unter Wasserausschluss bei Raumtemperatur vorgelegt. Dann wurde ein leichter Strom Wasserstoffgas bei atmosphärischem Druck durch die Mischung geleitet. Anschließend wurde auf 100 bis 110°C erhitzt und bei atmosphärischem Druck kontinuierlich Wasserstoffgas eingeleitet. Nach Ende der Freisetzung saurer Abgase (12 Stunden) wurde abgekühlt, der Katalysator durch Filtration abgetrennt und das Filtrat bei 27 mbar destilliert. Es wurde 3,5-Bis-(trifluormethyl)-benzylaldehyd mit einem Siedepunkt von 79°C in einer Ausbeute von 190,0 g erhalten. Das entspricht einer Ausbeute von 86 % der Theorie. Als Vorlauf fiel eine kleine Menge eines Gemisches aus Toluol und 3,5-Bis-(trifluormethyl)-benzol an.

### Beispiel 2

1 g 5 gew.-%iges Palladium auf Bariumsulfat (zurückgewonnen aus Beispiel 1) und 100 g 4-Trifluormethylbenzoylchlorid wurden unter Wasserausschluss bei Raumtemperatur vorgelegt. Dann wurde bei atmosphärischem Druck ein leichter Strom Wasserstoffgas eingeleitet. Anschließend wurde auf 100 bis 110°C erhitzt und bei atmosphärischem Druck kontinuierlich Wasserstoffgas eingeleitet. Nach Ende der Freisetzung saurer Abgase (6,5 Stunden) wurde auf Raumtemperatur abgekühlt, der Katalysator durch Filtration entfernt und das Filtrat bei 33 mbar destilliert. Mit einem Siedepunkt von 82 bis 86°C wurde 4-Trifluormethylbenzaldehyd in einer Ausbeute von 45,9 g erhalten. Das entspricht einer Ausbeute von 54 % der Theorie.

### Beispiel 3

0,5 g 5 gew.-%iges Palladium auf Bariumsulfat (zurückgewonnen aus Beispiel 1) und 48 g 3-Brom-4-trifluormethoxybenzoylchlorid wurden unter Wasserausschluss bei Raumtemperatur vorgelegt. Dann wurde bei atmosphärischem Druck ein leichter Strom Wasserstoffgas eingeleitet. Anschließend wurde auf 100 bis 110°C erhitzt und bei atmosphärischem Druck kontinuierlich Wasserstoffgas eingeleitet. Nach Ende der Freisetzung saurer Abgase (11 Stunden) wurde auf Raumtemperatur abgekühlt, der Katalysator durch Filtration entfernt und das Filtrat bei 25 mbar destilliert. Mit einem Siedepunkt von 112°C wurde 3-Brom-4-trifluormethoxybenzaldehyd in einer Ausbeute von 31,3 g enthalten. Das entspricht einer Ausbeute von 74 % der Theorie.

### Beispiel 4

0,5 g 5 gew.-%iges Palladium auf Bariumsulfat (zurückgewonnen aus Beispiel 1) und 50 g 3-Fluorbenzoylchlorid wurden unter Wasserausschluss bei Raumtemperatur vorgelegt. Dann wurde bei atmosphärischem Druck ein leichter Strom Wasserstoffgas eingeleitet. Anschließend wurde auf 80 bis 90°C erhitzt und bei atmosphärischem Druck kontinuierlich Wasserstoffgas eingeleitet. Nach Ende der Freisetzung saurer Abgase (9,5 Stunden) wurde auf Raumtemperatur abgekühlt, der Katalysator durch Filtration abgetrennt und das Filtrat bei 28 mbar destilliert. Mit einem Siedepunkt von 73°C wurde 3-Fluorbenzaldehyd in einer Ausbeute von 23,0 g erhalten. Das entspricht einer Ausbeute von 57 % der Theorie.

### Beispiel 5 (nicht erfindungsgemäß)

37 g 3,5-Bis-(trifluormethyl)-benzaldehyd und 4 g Raney-Nickel wurden bei Raumtemperatur in 150 ml Toluol vorgelegt. 30 bar Wasserstoffgas wurden aufgedrückt und die Mischung 7,5 Stunden bei 50°C unter Rühren hydriert. Anschließend wurde auf Raumtemperatur abgekühlt, entspannt und der Katalysator abfiltriert. Das Filtrat wurde eingeengt und das so entstandene Rohprodukt bei 17 mbar destilliert. Es wurde 3,5-Bis-(trifluormethyl)-benzylalkohol mit einem Siedepunkt von 97°C in einer Ausbeute von 31,5 g und einer Reinheit von 97,7 % erhalten. Das entspricht einer Ausbeute von 82,5 % der Theorie.

## Patentansprüche

1. Verfahren zur Herstellung Fluor enthaltender Benzaldehyde der Formel 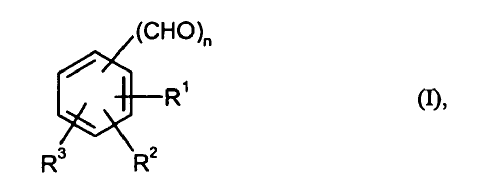 in der
n für 1 oder 2 steht und
R¹, R² und R³ unabhängig voneinander jeweils für Wasserstoff, Fluor, Chlor, Brom, C₁-C₆-Alkyl, C₁-C₆-Fluoralkyl, C₁-C₆-Fluoralkoxy oder C₁-C₆-Fluoralkylthio stehen,
wobei mindestens einer der Reste R¹ bis R³ für Fluor oder einen Fluor enthaltenden Rest steht und nicht mehr als zwei der Reste R¹ bis R³ für Brom, C₁-C₆-Fluoralkoxy und/oder C₁-C₆-Fluoralkylthio stehen,
gefunden, **dadurch gekennzeichnet, dass** man ein aromatisches Säurechlorid der Formel 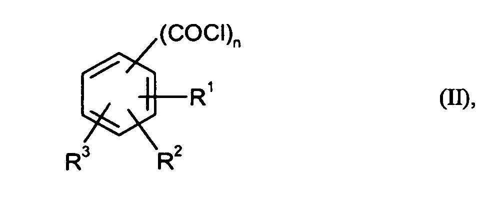 in der die verwendeten Symbole die bei Formel (I) angegebene Bedeutung haben,
mit Wasserstoff in Gegenwart eines geträgerten Palladium-Katalysators und eines Katalysator-Moderators in Abwesenheit eines Lösungsmittels umsetzt.

2. Verfahren nach Anspruch 1, **dadurch gekennzeichnet, dass** in den Formeln (I) und (II) die Reste R¹ bis R³ unabhängig voneinander für H, F, Cl, Br, CH₃, C₂H₅, CF₃, CF₂CH₃, C₂F₅, OCF₃ oder SCF₃ stehen, wobei mindestens einer der Reste R¹ bis R³ für F, CF₃, CF₂CH₃, C₂F₅, OCF₃ oder SCF₃ und höchstens einer der Reste R¹ bis R³ für Brom steht.

3. Verfahren nach Ansprüchen 1 und 2, **dadurch gekennzeichnet, dass** der Wasserstoff mit Drucken im Bereich 0,5 bis 3 bar und eine Reaktionstemperatur von 20 bis 200°C angewendet wird.

4. Verfahren nach Ansprüchen 1 bis 3, **dadurch gekennzeichnet, dass** der geträgerte Palladium-Katalysator als Trägermaterial Kohlen, Aluminiumoxide, Silikate, Kieselsäuren oder Bariumsulfat und 1 bis 10 Gew.-% Palladium enthält.

5. Verfahren nach Ansprüchen 1 bis 4, **dadurch gekennzeichnet, dass** das Gewichtsverhältnis von geträgertem Palladium-Katalysator zu eingesetztem aromatischem Säurechlorid bei 1:2 bis 1:1000 liegt.

6. Verfahren nach Ansprüchen 1 bis 5, **dadurch gekennzeichnet, dass** als Katalysator-Moderatoren organische Schwefelverbindungen eingesetzt werden.

7. Verfahren nach Ansprüchen 1 bis 6, **dadurch gekennzeichnet, dass** das Gewichtsverhältnis von Katalysator-Moderator zu geträgertem Palladium-Katalysator 1:1 bis 1:500 beträgt.

8. Verfahren nach Ansprüchen 1 bis 6, **dadurch gekennzeichnet, dass** man den Katalysator nach beendeter Umsetzung abtrennt und erneut wiederverwendet ohne erneuerten Zusatz von Katalysator-Moderator.

9. Verfahren nach Ansprüchen 1 bis 7, **dadurch gekennzeichnet, dass** man als Hilfsstoff bis zu 2,5 ml eines aromatischen Kohlenwasserstoffs, eines Halogenkohlenwasserstoffs, eines Halogenaromaten, eines aprotischen Amids, eines gegebenenfalls cyclischen Ethers oder eines Sulfons, bezogen auf 100 g eingesetztes aromatisches Säurechlorid verwendet.

10. Verfahren nach Ansprüchen 1 bis 9, **dadurch gekennzeichnet, dass** man den hergestellten Fluor enthaltenden Benzaldehyd nach Beendigung der Reaktion, gegebenenfalls nach Abtrennung des Katalysators, durch Destillation, gegebenenfalls bei vermindertem Druck, gewinnt.

## Claims

1. Process for preparing fluorine-containing benzaldehydes of the formula 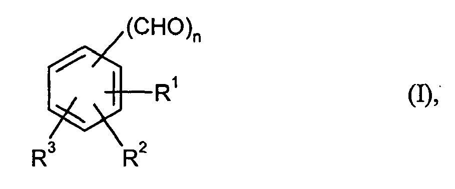 where
n represents 1 or 2 and
R¹, R² and R³ each represent, independently of one another, hydrogen, fluorine, chlorine, bromine, C₁-C₆-alkyl, C₁-C₆-fluoroakyl, C₁-C₆-fluoroalkoxy or C₁-C₆-iluoroalkylthio,
where at least one of the radicals R¹ to R³ represents fluorine or a fluorine-containing radical and not more than two of the radicals R¹ to R³ represents bromine, C₁-C₆-fluoroalkoxy and/or C₁-C₆-fluoroalkylthio,
**characterized in that** an aromatic acid chloride of the formula 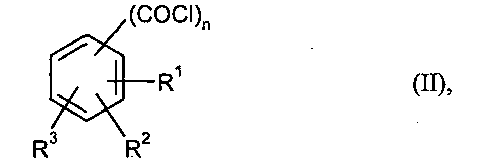 where the symbols used are as defined for formula (I),
is reacted with hydrogen in the presence of a supported palladium catalyst and a catalyst moderator in the absence of a solvent.

2. Process according to Claim 1, **characterized in that** the radicals R¹ to R³ in the formulae (I) and (II) each represent, independently of one another, H, F, Cl, Br, CH₃, C₂H₅, CF₃, CF₂CH₃, C₂F₅, OCF₃ or SCF₃, where at least one of the radicals R¹ to R³ represents F, CF₃, CF₂CH₃, C₂F₅, OCF₃ or SCF₃ and not more than one of the radicals R¹ to R³ represents bromine.

3. Process according to Claim 1 or 2, **characterized in that** the hydrogen is employed at pressures in the range from 0.5 to 3 bar and a reaction temperature of from 20 to 200°C.

4. Process according to Claims 1 to 3, **characterized in that** the supported palladium catalyst comprises carbon, aluminium oxides, silicates, silicas or barium sulphate as support material and from 1 to 10% by weight of palladium.

5. Process according to one of Claims 1 to 4, **characterized in that** the weight ratio of the supported palladium catalyst to the aromatic acid chloride used is from 1:2 to 1:1000.

6. Process according to one of Claims 1 to 5, **characterized in that** the catalyst moderator used comprises organic sulphur compounds.

7. Process according to one of Claims 1 to 6, **characterized in that** the weight ratio of catalyst moderator to the supported palladium catalyst is from 1:1 to 1:500.

8. Process according to one of Claims 1 to 6, **characterized in that** the catalyst is separated off after the reaction is complete and is reused without further addition of catalyst moderator.

9. Process according to one of Claims 1 to 7, **characterized in that** up to 2.5 ml of an aromatic hydrocarbon, a halogenated hydrocarbon, a halogenoaromatic, an aprotic amide, an acyclic or cyclic ether or a sulphone are used as auxiliary per 100 g of aromatic acid chloride used.

10. Process according to one of Claims 1 to 9, **characterized in that** the fluorine-containing benzaldehyde prepared is isolated after the reaction is complete, optionally after separating off the catalyst, by distillation, optionally under reduced pressure.

## Revendications

1. Procédé de préparation de benzaldéhydes fluorés de formule 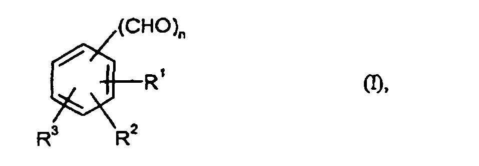 dans laquelle
n vaut 1 ou 2, et
R¹, R² et R³ représentent chacun, indépendamment les uns des autres, un atome d'hydrogène, un atome de fluore, un atome de chlore, un atome de brome, un groupe alkyle en C₁-C₆, un groupe fluoroalkyle en C₁-C₆, un groupe fluoroalcoxy en C₁-C₆ ou un groupe fluoroalkylthio en C₁-C₆,
où au moins l'un des radicaux R¹ à R³ représentent un radical fluoré et pas plus de deux des radicaux R¹ à R³ représentent un atome de brome, un groupe fluoroalcoxy en C₁-C₆ et/ou un groupe fluoroalkylthio en C₁-C₆,
**caractérisé en ce qu'**un chlorure d'acide aromatique de formule 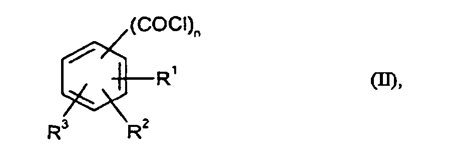 dans laquelle les symboles utilisés présentent la signification indiquée pour la formule (I),
est mis à réagir avec de l'hydrogène en présence d'un catalyseur au palladium supporté et d'un modérateur de catalyseur, en l'absence d'un solvant.

2. Procédé selon la revendication 1, **caractérisé en ce que** dans les formules (I) et (II), les radicaux R¹ à R³ représentent, indépendamment les uns des autres, un atome d'hydrogène, un atome de fluore, un atome de chlore, un atome de brome, un groupe CH₃, un groupe C₂H₅, un groupe CF₃, un groupe CF₂CH₃, un groupe C₂F₅, un groupe OCF₃ ou un groupe SCF₃, où au moins l'un des radicaux R¹ à R³ représente un atome de fluore, un groupe CF₃, un groupe CF₂CH₃, un groupe C₂F₅, un groupe OCF₃ ou un groupe SCF₃, et au plus l'un des radicaux R¹ à R³ représente un atome de brome.

3. Procédé selon les revendications 1 et 2, **caractérisé en ce que** l'hydrogène est employé avec des pressions dans la plage de 0,5 à 3 bars et une température de réaction de 20 à 200°C.

4. Procédé selon les revendications 1 à 3, **caractérisé en ce que** le catalyseur au palladium supporté comprend, en tant que matière de support, des charbons, des oxydes d'aluminium, des silicates, des silices ou du sulfate de baryum, et de 1 à 10 % en poids de palladium.

5. Procédé selon les revendications 1 à 4, **caractérisé en ce que** le rapport pondéral entre le catalyseur au palladium supporté et le chlorure d'acide aromatique utilisé est de 1 : 2 à 1 : 1000.

6. Procédé selon les revendications 1 à 5, **caractérisé en ce que** l'on utilise des composés soufrés organiques en tant que modérateurs de catalyseurs.

7. Procédé selon les revendications 1 à 6, **caractérisé en ce que** le rapport pondéral entre le modérateur de catalyseur et le catalyseur au palladium supporté est de 1 : 1 à 1 : 500.

8. Procédé selon les revendications 1 à 6, **caractérisé en ce que** le catalyseur est séparé à la fin de la réaction et de nouveau utilisé sans addition renouvelée de modérateur de catalyseur.

9. Procédé selon les revendications 1 à 7, **caractérisé en ce que** l'on utilise, en tant qu'auxiliaire, jusqu'à 2,5 ml d'un hydrocarbure aromatique, d'un hydrocarbure halogéné, d'un composé halogéno-aromatique, d'un amide aprotique, d'un éther éventuellement cyclique ou d'une sulfone, par rapport à 100 g de chlorure d'acide aromatique utilisé.

10. Procédé selon les revendications 1 à 9, **caractérisé en ce que** le benzaldéhyde fluoré préparé est obtenu par distillation, éventuellement sous pression réduite, après l'achèvement de la réaction, éventuellement après la séparation du catalyseur.
